# FASCICULE DE BREVET EUROPEEN

(11) **EP 2 903 589 B1**
(45) Date de publication et mention de la délivrance du brevet: **18.04.2018**
(21) Numéro de dépôt: 13766322.5
(22) Date de dépôt: 24.09.2013
(51) Int. Cl.: A61K 8/73, A61K 8/02, A61Q 19/08, A61K 8/24, A61K 47/12, A61K 9/08

(54) **FORMULATION AQUEUSE STÉRILE INJECTABLE À BASE D'ACIDE HYALURONIQUE RÉTICULÉ ET D'HYDROXYAPATITE POUR USAGE ESTHÉTIQUE**
INJIZIERBARE STERILE WÄSSRIGE FORMULIERUNG AUF DER BASIS VON VERNETZTER HYALURONSÄURE UND HYDROXYAPATIT ZUR THERAPEUTISCHEN VERWENDUNG
INJECTABLE STERILE AQUEOUS FORMULATION BASED ON CROSSLINKED HYALURONIC ACID AND HYDROXYAPATITE FOR AESTHETIC USE

(30) Priorité: 08.10.2012 FR 1259577
(43) Date de publication de la demande: 12.08.2015
(73) Titulaire: Anteis SA, 1228 Plan-Les-Ouates (CH)
(72) Inventeur: GAVARD MOLLIARD, Samuel, F-74250 Bogève (FR)
(74) Mandataire: Ricker, Mathias
(86) Numéro de dépôt international: PCT/EP2013/069874
(87) Numéro de publication internationale: WO 2014/056722

(56) Documents cités:
- WO-A1-2004/011053
- FR-A1- 2 938 187
- FR-A1- 2 945 949
- Kristoffer Bergman: "Hyaluronan Derivatives and Injectable Gels for Tissue Engineering" In: "Hyaluronan Derivatives and Injectable Gels for Tissue Engineering", 14 novembre 2008 (2008-11-14), Universitetbiblioteket, Uppsala, Sweden, XP055070428, ISBN: 978-9-15-547335-8 vol. 573 le document en entier
- BAKOS D ET AL: "Hydroxyapatite-collagen-hyaluronic acid composite", BIOMATERIALS, ELSEVIER SCIENCE PUBLISHERS BV., BARKING, GB, vol. 20, no. 2, 1 janvier 1999 (1999-01-01), pages 191-195, XP004168893, ISSN: 0142-9612, DOI: 10.1016/S0142-9612(98)00163-X

## Description

### DOMAINE DE L'INVENTION

La présente invention a pour objet une formulation aqueuse stérile injectable, prête à l'emploi, résorbable utilisée dans des buts esthétiques sous forme de gel viscoélastique cohésif particulaire comprenant i) de l'acide hyaluronique réticulé, ou l'un de ses sels, à une concentration comprise entre 1% à 4% (masse/volume) ; la réticulation effectuée permettant d'obtenir un gel à base d'acide hyaluronique réticulé de structure dite cohésive, et ii) de l'hydroxyapatite, à une concentration comprise entre 5% à 60% (masse/volume), ladite hydroxyapatite étant sous forme de particules de taille moyenne inférieure ou égale à 200 µm; ladite formulation aqueuse stérile injectable possédant des propriétés viscoélastiques telles que Tanδ à la fréquence de 1 Hz est inférieur ou égal à 0.60.

### CONTEXTE DE L'INVENTION

Le vieillissement est un phénomène naturel auquel tout individu est confronté. Il s'accompagne inévitablement d'une diminution de l'activité cellulaire du corps humain. Les signes les plus visibles de l'âge apparaissent au niveau du visage : la peau se détend et les premières rides apparaissent. De nombreuses solutions ont été développées pour tenter de ralentir l'apparition des ces signes de l'âge ou de réparer les signes déjà installés.

Parmi ces solutions, on peut mentionner une technique qui consiste à injecter dans ou sous la peau des substances dites de comblement, ces substances pouvant être résorbables ou non résorbables in vivo. Le rôle de ces substances est de combler les parties affaissées en créant un volume dans ou sous la peau, pour traiter différentes parties du corps, en particulier le visage. Par cet effet mécanique, la peau est retendue et les rides lissées, conduisant à un aspect plus jeune de la zone traitée.

Parmi les substances de comblement dites résorbables, on peut citer l'acide hyaluronique (AH) réticulé (également appelé « AH stabilisé ») utilisé en dermo-esthétique. Il est injecté dans ou sous le derme pour combler les rides ou restaurer le volume de différentes zones du corps pour une période de plusieurs mois. Il présente l'avantage de présenter très peu d'effets secondaires en post d'injection et des complications extrêmement rares sur le long terme. D'autre part, en cas de mauvaise injection, le praticien à la possibilité de corriger son traitement en injectant une solution de hyaluronidases (enzymes spécifiques de l'AH), solution qui va dégrader le produit à base de AH réticulé préalablement injecté. Les injections de AH réticulé, du fait de leur disparition progressive (résorption du polymère dans les tissus au cours du temps), doivent être répétées à intervalles réguliers, généralement de 6 à 12 mois, afin de maintenir l'efficacité du traitement. L'acide hyaluronique non réticulé, lui, à un temps de résidence court au niveau de la peau (demi-vie inférieure à une semaine), il est dégradé in vivo par différents facteurs comme la dégradation radicalaire, enzymatique, thermique et mécanique. C'est bien la réticulation qui permet de significativement accroître sa demi-vie en ralentissant les cinétiques de dégradation de l'acide hyaluronique selon les facteurs décrits ci-dessus, permettant ainsi une efficacité du traitement esthétique pouvant atteindre environ 12 mois.
Une recherche scientifique intensive est effectuée à l'échelle mondiale afin de mettre au point des traitements à base d'acide hyaluronique possédant une performance renforcée dans le temps. L'objectif étant notamment d'avoir des produits capable de se dégrader moins rapidement afin de conserver un effet esthétique optimal sur une période la plus longue possible, tout en conservant un très haut niveau de sécurité des produits injectés.

D'autres substances de comblement résorbables existent sur le marché de la dermo-esthétique. On peut citer par exemple les produits contenant des particules d'hydroxyapatite de calcium. Ces particules sont suspendues dans une phase aqueuse pouvant contenir un polymère comme la carboxymethylcellulose, un dérivé de la cellulose. Les produits de cette famille sont injectés dans ou sous le derme pour combler les rides ou restaurer le volume de différentes zones du corps et en particulier le visage. Ils possèdent un haut niveau de biocompatibilité, ce qui justifie l'absence d'un test d'allergie préalable à l'injection. Pour ces produits, peu d'effets secondaires ou de complications sont reportés et une durée d'efficacité de l'ordre de 12 mois ou plus est observée. Du point de vue de la résorption, la phase aqueuse est rapidement éliminée de la zone traitée et les particules d'hydroxyapatite sont dégradées et métabolisées par les macrophages au cours du temps.
Une stimulation de la production endogène de collagène par les particules d'hydroxyapatite est également décrite pour ces produits injectés dans les tissus de la peau.
Ces produits ont malheureusement tendance à migrer, comme décrit dans différentes communications scientifiques. Cette migration pose un problème car elle induit une perte prématurée de l'effet esthétique (moins de biomatériau au niveau de la zone corrigée) et peut potentiellement induire des effets secondaires (les particules peuvent notamment se concentrer dans certaines parties plus ou moins éloignées de la zone à traiter (du fait des contraintes mécaniques soumises au biomatériau) et induire localement des zones dites dures.

Dans ce contexte, il est important de mettre à la disposition des praticiens des formulations possédants des propriétés mécaniques remarquables adaptées aux injections à buts cosmétiques et/ou esthétiques ayant une longévité accrue dans les tissus, prêtes à l'emploi, et ne présentant pas les inconvénients décrits précédemment.

### RESUME DE L'INVENTION

L'invention concerne une formulation aqueuse stérile injectable et biorésorbable, utilisée dans des buts esthétiques, sous forme de gel viscoélastique cohésif particulaire comprenant i) de l'acide hyaluronique réticulé, ou l'un de ses sels, à une concentration comprise entre 1% à 4% (masse/volume) ; la masse moléculaire de l'acide hyaluronique, ou de l'un de ses sels, étant comprise entre 2.5x10⁵Da et 4x10⁶Da, la réticulation effectuée permettant d'obtenir un gel à base d'acide hyaluronique réticulé de structure dite cohésive, et ii) de l'hydroxyapatite, à une concentration comprise entre 5% à 60% (masse/volume), ladite hydroxyapatite étant sous forme de particules de taille moyenne inférieure ou égale à 200 µm; ladite formulation aqueuse stérile injectable possédant des propriétés viscoélastiques telles que Tanδ à la fréquence de 1 Hz est inférieur ou égal à 0.60.

Selon un autre objet, la présente invention concerne un procédé de préparation d'une formulation aqueuse stérile injectable comprenant les étapes consistant à : a) préparer un premier mélange comprenant au moins 1% à 4% en poids d'acide hyaluronique réticulé ou d'un de ses sels, par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite cohésive, b) purifier ledit premier mélange, c) ajouter ensuite l'hydroxyapatite à une concentration comprise entre 5% à 60% (masse/volume) en la dispersant de manière homogène dans le gel à base d'acide hyaluronique réticulé, d) mettre le gel ainsi obtenu sous forme prête à l'emploi, e) stériliser le produit à la chaleur humide.

Selon un autre objet également, la présente invention concerne un kit se présentant de préférence sous la forme de seringue contenant la formulation telle que décrite précédemment.

### BREVE DESCRIPTION DE LA FIGURE

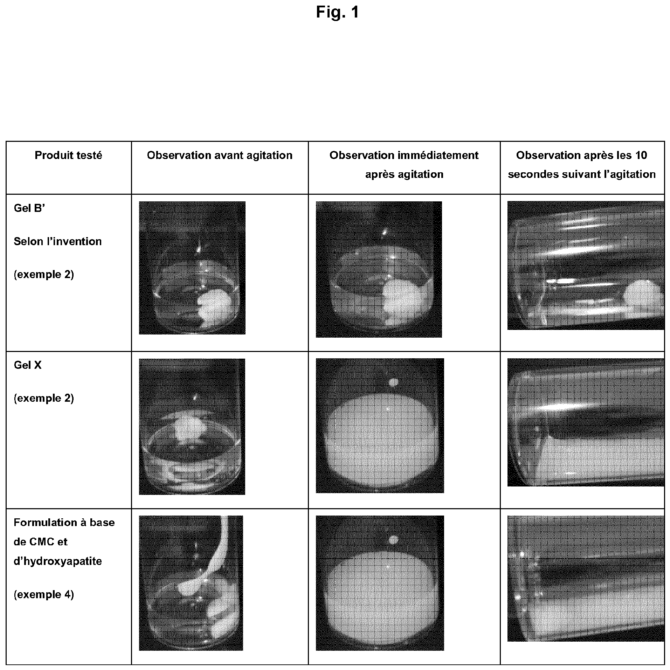

**La** **figure 1** représente des photos de la comparaison des gels B', X et de la formulation à base de CMC et d'hydroxyapatite, selon le test décrit dans l'exemple 2.

### DESCRIPTION DETAILLEE DE L'INVENTION

L'invention décrite ci-après a pour objectif de proposer une nouvelle formulation aqueuse stérile injectable biorésorbable utilisée dans des buts cosmétiques et esthétiques et présentant des propriétés spécifiques de viscoélasticité, de comblements et de performance à long terme. Cette formulation est caractérisée en ce qu'elle est sous forme de gel viscoélastique cohésif particulaire comprenant
i) de l'acide hyaluronique réticulé, ou l'un de ses sels, à une concentration comprise entre 1% à 4% (masse/volume) ; la réticulation effectuée permettant d'obtenir un gel à base d'acide hyaluronique réticulé de structure dite cohésive, et
ii) de l'hydroxyapatite, à une concentration comprise entre 5% à 60% (masse/volume), ladite hydroxyapatite étant sous forme de particules de taille moyenne inférieure ou égale à 200 µm;
ladite formulation aqueuse stérile injectable possédant des propriétés viscoélastiques telles que Tanδ à la fréquence de 1 Hz est inférieur ou égal à 0.60.

De manière tout à fait surprenante, il a été constaté que cette formulation possède une capacité remarquable à créer du volume dans les tissus sur le long terme, ceci grâce à une synergie entre l'acide hyaluronique réticulé et les particules d'hydroxyapatite, selon les conditions de l'invention.
D'un point de vue mécanique, les particules d'hydroxyapatite (de comportement solide : forte élasticité et viscosité négligeable) renforcent considérablement l'élasticité du gel et donc sa capacité à créer du volume en induisant une force/pression importante sur les tissus pour corriger la zone déficiente à traiter.

L'acide hyaluronique réticulé, lui, apporte des propriétés de viscoélasticité, c'est-à-dire d'élasticité mais également de viscosité permettant d'avoir une consistance de gel se rapprochant de celle des tissus et donc ainsi de contrebalancer la très forte élasticité et l'absence de viscosité apportée par les particules d'hydroxyapatite. Ceci permettant d'avoir un produit s'intégrant dans les tissus de manière beaucoup plus homogène (le patient ressentant moins le produit à la palpation), moins traumatique pour les tissus (forte limitation de l'inflammation en post injection) et moins douloureux à l'injection.
D'autre part, l'acide hyaluronique réticulé, dans les conditions de l'invention, va permettre de réduire considérablement la migration des particules d'hydroxyapatite, particules qui sont retenues au sein du gel, de part la forte cohésivité apportée par l'acide hyaluronique réticulé de nature cohésive (acide hyaluronique réticulé possédant une faible cinétique de résorption). Cette forte limitation de la migration permet d'avoir un gel avec une capacité volumatrice améliorée sur le long terme et de réduire les effets secondaires comme l'apparition de zones dites dures, ressenties par le patient.

L'acide hyaluronique est un polysaccharide composé de la répétition d'unités disaccharide de glucuronate et de N-acetyl glucosamine. Il est réparti largement parmi les tissus conjonctifs, épithéliaux et nerveux chez les humains comme chez les animaux. Il constitue l'un des principaux composants de la matrice extracellulaire. Il contribue de façon significative à la prolifération et à la migration des cellules. Il est notamment trouvé en concentration importante dans l'humeur aqueuse, le liquide synovial, la peau et le cordon ombilical.
Parmi les sels d'acide hyaluronique préférés selon l'invention, on citera les sels d'acide hyaluronique, avec un cation, par exemple un sel mono- ou divalent tel que les sels de sodium, potassium, magnésium, calcium, manganèse. Les sels de sodium sont tout particulièrement préférés.

Selon l'invention, l'acide hyaluronique ou d'un de ses sels, est sous forme réticulée. Cette réticulation est obtenue par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite cohésive, encore appelée monophasique.

La nature cohésive du gel à base d'acide hyaluronique réticulé est une spécificité majeure et nécessaire de l'invention. Le gel ne doit pas se désagréger rapidement lorsqu'on l'introduit dans de l'eau, comme le fait un gel de nature non cohésive, encore appelé gel biphasique (type de gel à base d'acide hyaluronique réticulé n'étant pas en mesure de maintenir les particules d'hydroxyapatite et donc d'éviter la migration). L'exemple 2 met en avant cette différence entre un gel cohésif et un gel non cohésif.

La présente invention comprend généralement une concentration en acide hyaluronique réticulé, ou de l'un de ses sels, comprise entre 1% à 4% (masse/volume), de préférence entre 1% et 3% (masse/volume). Selon une variante particulièrement préférée, la concentration en acide hyaluronique réticulé, ou de l'un de ses sels, est comprise entre 1.5% à 2.5% (masse/volume). Alternativement, la concentration en acide hyaluronique réticulé, ou de l'un de ses sels, peut être comprise entre 1.5% à 3% (masse/volume), ou 1% à 2.5% (masse/volume).

Avantageusement, la formulation aqueuse selon l'invention comprend de l'acide hyaluronique, ou l'un de ses sels, dont la masse moléculaire est préférablement comprise entre 2.5x10⁵ Da et 4x10⁶ Da. Selon une variante particulièrement préférée, cette masse moléculaire est comprise entre 1x10⁶ Da et 3x10⁶. Alternativement, la masse moléculaire est comprise entre 1x10⁶ Da et 2.5x10⁶, ou 2.5x10⁵ Da et 3x10⁶ Da.

L'hydroxyapatite est une espèce minérale de la famille des phosphates, de formule Ca5(PO4)3(OH), usuellement écrite Ca10(PO4)6(OH)2 pour souligner le fait que la maille de la structure cristalline comprend 2 molécules. L'hydroxyapatite appartient à la famille cristallographique des apatites, composés isomorphes possédant une même structure hexagonale. Ce composé est utilisé comme biomatériau depuis de nombreuses années dans différentes spécialités médicales.

La présente invention comprend généralement une concentration en particules d'hydroxyapatite comprise entre 5 à 60% (masse/volume), de préférence entre 10 à 50% (masse/volume), de préférence entre 20 à 40% (masse/volume) et la taille moyenne des particules d'hydroxyapatite est inférieure ou égale à 200 µm, de préférence inférieure à 50 µm, de préférence supérieur à 10 µm.

Il a été observé que les propriétés de viscosité et d'élasticité de la formulation selon invention sont optimales lorsque le paramètre Tan delta ou Tanδ, correspondant au rapport [module visqueux G"/module élastique G'] à la fréquence de 1 Hz, est inférieur ou égal à 0.60, de préférence inférieur ou égal à 0.58. En effet, il a été montré que le caractère élastique de la formulation selon l'invention, par rapport à sa viscosité, doit être suffisamment important pour pouvoir éviter la sédimentation des particules d'hydroxyapatite. Ainsi, il a été observé qu'en dessus de 0.60, les particules d' hydroxyapatite ont tendance à sédimenter au cours du temps. Cette sédimentation implique l'obtention d'une formulation à base de particules d'hydroxyapatite non homogène, ce qui n'est pas satisfaisant d'une part pour l'acte d'injection de la formulation à travers l'aiguille (bouchage d'aiguille) et d'autre part pour la sécurité et la performance de la formulation au niveau de la zone d'injection (par exemple, création de zones dites dures dans les tissus de la peau).
En général, la mesure de l'élasticité (G') et du rapport de la viscosité sur l'élasticité (Tan delta=G"/G') est effectuée en réalisant un balayage en fréquence de 0.01 à 100 Hz à l'aide d'un rhéomètre avec une géométrie plate de 40 mm, un entrefer de 1000 micromètres et une température d'analyse de 25°C.

Comme montré dans l'exemple 2, la cohésivité de la formulation selon l'invention est un élément majeur mais il est également nécessaire que le caractère viscoélastique de celui-ci soit approprié afin :
- d'éviter la sédimentation au cours du temps des particules d'hydroxyapatite au sein de leur contenant, et
- d'éviter d'avoir un produit qui va se séparer en 2 phases (particules d'hydroxyapatite et gel d'acide hyaluronique réticulé) lors de l'injection et/ou au niveau de la zone d'injection, créant ainsi des hétérogénéités au niveau de la zone traitée.

L'invention a également pour objectif de présenter une meilleure longévité au cours du temps en comparaison avec les formulations de l'art antérieur. Cette meilleure longévité de l'effet esthétique est obtenue grâce à la capacité de l'acide hyaluronique réticulé à maintenir sur le long terme les particules d'hydroxyapatite au niveau de la zone d'injection et aux particules d'hydroxyapatite à conférer des propriétés mécaniques/rhéologiques remarquables sur le long terme. Il sera donc moins souvent nécessaire de renouveler les injections avec la formulation selon l'invention, le gain sur la longévité en clinique étant probablement de plusieurs mois.

Il est également important de préciser que la présence des particules d'hydroxyapatite, radio-opaques, confère un avantage au gel car pouvant être localisé aisément par le praticien sous radiographie pendant et/ou après l'injection.

D'autre part, la capacité des particules d'hydroxyapatite à stimuler la production endogène de collagène est un élément important de l'invention. La résorption lente du produit dans les tissus va s'accompagner d'une production de collagène (création de volume et d'élasticité), qui va permettre de participer à la performance du traitement sur le long terme.

La possibilité donnée au praticien d'injecter une solution de hyaluronidases pour corriger son injection et dégrader l'acide hyaluronique réticulé composant le produit confère également un avantage à l'invention. Cette injection ne permet néanmoins pas d'accélérer la résorption des particules d'hydroxyapatite : il n'y a donc pas dégradation complète du produit au sein des tissus.

La présente invention consiste donc en une formulation, telle que décrite ci-dessus, utilisée pour le comblement et/ou la restauration de volumes et/ou le remplacement de tissus biologiques, en particulier i) restauration des volumes du visage (joues, menton, pommettes, tempes, ...), ii) restauration des volumes du corps (fesses, seins, mains...), iii) restauration des volumes du visage chez des patients HIV atteint de lipodystrophie faciale.

La formulation selon l'invention est généralement utilisée telle quelle mais il n'est pas exclu qu'il lui soit ajouté au moins un autre additif (autre que ceux citées plus haut) et/ou au moins un principe actif.

De manière avantageuse, la formulation selon l'invention est « une formulation prête à l'emploi », car le praticien n'a pas à mélanger lui-même l'acide hyaluronique réticulé et une solution d'hydroxyapatite, juste avant leur injection.

Ainsi, la formulation peut comprendre en outre un ou plusieurs matériaux céramiques. Ces matériaux sont généralement choisis parmi le groupe comprenant le phosphate tricalcique, le carbonate de calcium et le sulfate de calcium, ou une combination de plusieurs de ses matériaux céramiques.

La formulation selon l'invention peut également comprendre en outre un ou plusieurs anesthésiques, choisi parmi le groupe comprenant la lidocaïne seule ou en combinaison avec de l'adrénaline, la procaïne, l'étidocaïne seule ou en combinaison avec de l'adrénaline, l'articaïne seule ou en combinaison avec de l'adrénaline, la mépivacaïne, la pramocaïne, la quinisocaïne, ou un ou plusieurs des sels de ces anesthésiques. Selon une variante particulièrement préférée, l'anesthésique choisi est le chlorhydrate de lidocaïne. La présence d'un anesthésique dans la formulation selon l'invention présente un intérêt majeur pour l'amélioration du confort du patient au cours et après injection.

Selon un autre mode particulier de l'invention, la formulation selon l'invention peut également comprendre en outre un ou plusieurs antioxydants, comme les antioxydants de la famille des polyols. L'antioxydant pourra être choisi parmi le groupe des polyols comprenant le sorbitol, le glycérol, le mannitol ou le propylène glycol.

Selon un autre objet, la présente invention concerne un procédé de préparation d'une formulation aqueuse stérile injectable comprenant les étapes consistant à : a) préparer un premier mélange comprenant au moins 1% à 4% en poids d'acide hyaluronique réticulé ou d'un de ses sels, par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite cohésive, b) purifier ledit premier mélange, c) ajouter ensuite l'hydroxyapatite à une concentration comprise entre 5% à 60% (masse/volume) en la dispersant de manière homogène dans le gel à base d'acide hyaluronique réticulé, d) mettre le gel ainsi obtenu sous forme prête à l'emploi, e) stériliser le produit à la chaleur humide.

La stérilisation de la formulation selon l'étape e) est réalisée à la chaleur humide. L'homme de l'art saura sélectionner un cycle de stérilisation à la chaleur (température et durée du cycle de stérilisation) approprié à la stérilisation de son produit. Par exemple, les cycles de stérilisation à la chaleur humide suivants peuvent être utilisés : 131°C, 1 min / 130°C, 3 min / 125°C, 7 min / 121°C, 20 min.

Selon un autre objet, la présente invention concerne un kit se présentant de préférence sous la forme de seringue contenant la formulation telle que décrit précédemment.

La présente invention concerne également un kit sous forme d'un contenant différent d'une seringue comme une ampoule ou un flacon contenant la formulation telle que décrit précédemment.

L'inventeur a montré dans les exemples suivants que la formulation selon l'invention à base de HA réticulé doit posséder des propriétés spécifiques, notamment de cohésivité. Si tel n'est pas le cas (voir les exemples avec le HA non réticulé ou avec un HA réticulé ne possédant pas la structure revendiquée), les particules d'hydroxyapatite ne sont pas maintenues correctement au sein de la matrice et peuvent donc diffuser relativement facilement hors du gel, ce qui implique une perte de volume au niveau de la zone traité (c'est-à-dire une perte d'efficacité) et des possibles complications du fait de cette migration entrainant des problèmes de sécurité.

L'invention va maintenant être illustrée à titre non limitatif par les exemples 1 à 4 suivants :

### EXEMPLES

### Exemple 1

### Préparation d'un gel à base d'acide hyaluronique réticulé de structure dite cohésive

**Etape 1 :** 3.5 g de hyaluronate de sodium de poids moléculaire 2,6 MDa sont ajoutés à de la soude 1 % (30,5 g). Le mélange est laissé à l'homogénéisation pendant 1 h 30. 420 mg de butanediol diglycidyl ether (BDDE) sont ajoutés au mélange qui est homogénéisé, fermé puis placé au bain-marie à 50°C pendant 2 h. Le mélange est ensuite neutralisé par l'ajout de 7.5 g de HCl 1N.

Le gel est purifié pendant 24h par dialyse avec une solution physiologique iso-osmolaire et possédant un pH neutre (cellulose régénérée, limite de séparation : masse moléculaire = 60 kDa) afin d'obtenir une concentration en acide hyaluronique de 25 mg/ml (2.5%). Il est ensuite homogénéisé dans un mélangeur classique à pâles pendant 1h30 (= gel A1 / 124 g).

Le gel peut enfin être dégazé, rempli en seringues en verre de 2 ml et stérilisé par autoclavage vapeur à 130°C pendant 3 minutes (= gel A / gel viscoélastique de structure dite cohésive ou monophasique).

**Etape 2 :** préparation du gel selon l'invention. Dans 100g de gel A1, on ajoute 42.9 g de phosphocalcium hydroxyapatite Ca10(PO4)6(OH)2 dont les particules ont une granulométrie moyenne comprise entre 30 et 50 micromètres puis on homogénéise le gel dans un mélangeur classique à pâles pendant 1h30 (= gel B1 / 142.9g).
Le gel peut enfin être dégazé, rempli en seringues en verre de 2 ml et stérilisé par autoclavage vapeur à 130°C pendant 3 minutes (= gel B).
Le gel est viscoélastique cohésif particulaire. En effet, celui-ci se présente sous la forme d'un gel viscoélastique (il présente des propriétés d'élasticité G' et de viscosité G" / voir ci-dessous), possédant une forte cohésivité (voir exemple 2) et contenant des particules d'hydroxyapatite.
La concentration en acide hyaluronique du gel est de 17.5 mg/ml (1.75%) (dosage au carbazole, méthode de la Pharmacopée Européenne). D'autre part, le pH (7.15) et l'osmolarité (315 mOsm/kg) du gel sont physiologiques.
Le gel est facilement injectable à travers une aiguille : Une force de 26.3 N est nécessaire pour pousser le gel à travers une aiguille de 21G 1 1/2, en considérant une vitesse de poussée de 12.5 mm/minute.

Les gels A et B sont caractérisés d'un point de vue mécanique/rhéologique : Le rhéomètre utilisé pour effectuer ces caractérisations est un AR2000 (TA instruments) avec une géométrie plate de 40 mm, un entrefer de 1000 micromètres est une température d'analyse de 25°C.

Une mesure de l'élasticité (G') et du rapport de la viscosité sur l'élasticité (Tan delta=G"/G') est effectuée en réalisant un balayage en fréquence de 0.01 à 100 Hz. Une comparaison des paramètres est effectuée à 1 Hz.

| **Gel** | **G'(1 Hz) en Pa** | **Tan delta (1 Hz)** |
|---|---|---|
| A | 184 | 0.25 |
| B | 381 | 0.29 |

On constate que le produit B possède une élasticité significativement plus forte que le produit A. Le Tan delta des 2 produits A et B, quant à lui, est relativement proche : le gel B conserve un caractère visqueux important, malgré la présence des particules d'hydroxyapatite (qui elles possèdent une forte élasticité et une viscosité négligeable). Cette plus forte élasticité, en combinaison avec la forte cohésivité du produit selon l'invention, confère une meilleure capacité du produit à créer du volume dans les tissus.

Une mesure de la force normale induite par le gel à tester est effectuée par compression de l'échantillon entre le plan peltier et la géométrie pour un entrefer de 1500 micromètres et une quantité de gel de 1.4 g.

| **Gel** | **Force normale (N)** |
|---|---|
| A | 0.86 |
| B | 1.47 |

On constate que le produit B possède une élasticité et une force normale induite significativement plus forte que le produit A.

Cette plus forte élasticité, en combinaison avec la forte cohésivité du gel selon l'invention, confère une meilleure capacité du produit à créer du volume dans les tissus.

### Exemple 2

### Importance de la structure dite cohésive du gel à base de HA réticulé - Comparatif

Le gel A1 (de structure dite cohésive ou monophasique) décrit dans l'exemple 1 est dialysé avec une solution physiologique iso-osmolaire et possédant un pH neutre (cellulose régénérée, limite de séparation : masse moléculaire = 60 kDa) afin d'obtenir une concentration en acide hyaluronique de 20 mg/ml (2%).
De l'hydroxyapatite de calcium est ensuite ajouté dans le gel afin d'obtenir une concentration de 200 mg/ml (20%) puis un mélange à la spatule est effectué (2 minutes pour 5g de gel).

Le gel ainsi obtenu est ensuite stérilisé à l'autoclave à 121°C pendant 20 minutes (= gel B' selon l'invention).

Le gel commercial Restylane® Perlane® (lot 11363-1) à base d'acide hyaluronique réticulé de structure dite non cohésive ou biphasique, dont la concentration en acide hyaluronique est de 20 mg/ml (2%) est dopé avec 200 mg/ml (20%) d'hydroxyapatite de calcium par mélange à la spatule (2 minutes pour 5g de gel).

Le gel ainsi obtenu est ensuite stérilisé à l'autoclave à 121°C pendant 20 minutes (= gel X).

Le gel A1 et le gel Restylane® Perlane® sont comparés selon le test suivant :
Dans des flacons en plastique de 30 ml contenant 5 ml d'eau purifiée, on introduit 1 ml de gel A1 dans le flacon 1 et 1 ml de gel Restylane® Perlane® dans le flacon 2. Après fermeture des flacons, on mélange manuellement les 2 flacons pendant 5 secondes.
Après 10 secondes, on observe que le gel Restylane® Perlane® s'est complètement désagrégé/dispersé sous forme d'une multitude de particules dans la solution aqueuse. Le gel Restylane® Perlane® possède donc bien une structure dite non cohésive ou biphasique (le gel se disperse rapidement dans la solution aqueuse).

Le gel A1, lui, est toujours sous la forme d'une « boule de gel » dans la solution aqueuse. Il possède donc bien une structure dite cohésive ou monophasique (le gel ne se disperse pas rapidement dans la solution aqueuse, il possède une forte cohésivité, contrairement au gel Restylane® Perlane®).

Le gel B' selon l'invention et le gel X sont comparés selon le test suivant (voir figure 1): Dans des flacons en plastique de 30 ml contenant 5 ml d'eau purifiée, on introduit 1 ml de gel B' dans le flacon 1 et 1 ml de gel X dans le flacon 2. Après fermeture des flacons, on mélange manuellement les 2 flacons pendant 5 secondes.
Après 10 secondes, on observe que le gel X s'est complètement désagrégé/dispersé sous forme d'une multitude de particules dans la solution aqueuse. Le gel X possède une structure viscoélastique non cohésive particulaire. Il ne correspond pas aux caractéristiques du gel selon l'invention. En pratique médicale pour une utilisation en esthétique, celui-ci va diffuser/migrer au niveau de la zone d'injection.

Le gel B', lui, est toujours sous la forme d'une « boule de gel » dans la solution aqueuse. Il possède donc bien une structure cohésive particulaire qui va permettre, dans le cadre de la pratique médicale pour une utilisation en esthétique, de ne pas diffuser/migrer au niveau de la zone injectée, et ainsi d'éviter les complications liées à la migration de particules d'hydroxyapatite dans les tissus mais également d'avoir une meilleure performance long terme du produit car le gel injecté va pouvoir maintenir sa capacité à créer du volume dans les tissus sur une longue période, du fait de l'absence de migration du biomatériau de la zone traitée.

### Exemple 3

### Importance de la viscoélasticité du gel selon l'invention - Comparatif

Soit C un gel préparé selon le même protocole (étapes 1&2) que celui décrit en exemple 1 en introduisant 200 mg de BDDE au lieu de 420 mg.
Soit D un gel préparé selon le même protocole (étapes 1&2) que celui décrit en exemple 1 en introduisant 290 mg de BDDE au lieu de 420 mg.

Le gel C est caractérisé d'un point de vue mécanique/rhéologique.
Le rhéomètre utilisé pour effectuer les caractérisations rhéologiques est un AR2000 (TA instruments) avec une géométrie plate de 40 mm, un entrefer de 1000 micromètres est une température d'analyse de 25°C.

Une mesure du rapport de la viscosité sur l'élasticité (Tan delta=G"/G') est effectuée en réalisant un balayage en fréquence de 0.01 à 100 Hz.
Une comparaison des paramètres est effectuée à 1 Hz.

| **Gel** | **Tan delta = G"/G'(1 Hz)** |
|---|---|
| C | 0.84 |
| D | 0.58 |

On constate que les particules d'hydroxyapatite ont tendance à sédimenter au cours du temps (phénomène bien observé par passage d'un échantillon à la centrifugeuse) pour le gel C, ce qui n'est pas observé pour le gel D.

Cette sédimentation implique l'obtention de produits à base de particules d'hydroxapatite non homogène, ce qui n'est pas satisfaisant pour l'acte d'injection du gel à travers l'aiguille (bouchage de l'aiguille) mais également pour la sécurité et la performance du produit au niveau de la zone d'injection (risques importants de complications comme par exemple la création de zones dites dures dans les tissus de la peau).

Comme montré dans l'exemple 2, la cohésivité du gel selon l'invention est importante mais il est également nécessaire que le caractère viscoélastique de celui-ci soit approprié afin :
- d'éviter la sédimentation au cours du temps des particules d'hydroxyapatite au sein de leur contenant
- d'éviter d'avoir un produit qui va se séparer en 2 phases (particules d'hydroxyapatite et gel d'acide hyaluronique réticulé) lors de l'injection et/ou au niveau de la zone d'injection, créant ainsi des hétérogénéités au niveau de la zone traitée.

Le caractère élastique du gel (par rapport à sa viscosité) doit donc être suffisamment important pour pouvoir éviter la sédimentation des particules.

### Exemple 4

### Comparaison d'un gel selon l'invention avec des solutions de l'art antérieur

### a) Formulation à base de HA non réticulé et d'hydroxyapatite

Comme décrit dans la littérature, in vivo, l'acide hyaluronique non réticulé a une demi-vie inférieure à une semaine.
Par conséquent, une solution de HA non réticulé avec hydroxyapatite n'est pas intéressante car l'acide hyaluronique non réticulé va se résorber très rapidement et il ne va pas permettre d'empêcher la migration des particules d'hydroxyapatite sur le long terme.

### b) Formulation aqueuse d'hydroxyapatite

Une solution aqueuse d'hydroxyapatite (S1) est préparée (30% de phosphocalcium hydroxyapatite possédant une granulométrie comprise entre 30 et 50 micromètres dans une solution physiologique iso-osmolaire et possédant un pH neutre).

Dans un flacon en plastique de 30 ml contenant 5 ml d'eau purifiée, on introduit 1 ml de la solution S1. On observe une dispersion immédiate des particules d'hydroxyapatite dans le flacon.

Contrairement à la formulation selon l'invention, la solution S1 n'est pas en mesure de maintenir les particules d'hydroxyapatite au niveau de la zone d'injection sur le long terme.

### c) Formulation à base de CMC et d'hydroxyapatite (voir figure 1).

Une formulation aqueuse de carboxyméthylcellulose (CMC) et d'hydroxyapatite (S3) est préparée (30% de phosphocalcium hydroxyapatite possédant une granulométrie comprise entre 30 et 50 micromètres, et 2% de CMC à 250 000 Da dans une solution physiologique iso-osmolaire et possédant un pH neutre).

Dans un flacon en plastique de 30 ml contenant 5 ml d'eau purifiée, on introduit 1 ml de la formulation S3. Après fermeture du flacon, on mélange manuellement le flacon pendant 5 secondes.

Après 10 secondes, on observe que la formulation S3 s'est complètement désagrégée/dispersée sous forme d'une multitude de particules dans la solution aqueuse.

Contrairement à la formulation selon l'invention, la formulation S3 n'est pas en mesure de maintenir les particules d'hydroxyapatite au niveau de la zone d'injection sur le long terme.

## Revendications

1. Formulation aqueuse stérile injectable, utilisée dans des buts esthétiques, sous forme de gel viscoélastique cohésif particulaire comprenant
i) de l'acide hyaluronique réticulé, ou l'un de ses sels, à une concentration comprise entre 1% à 4% (masse/volume); la réticulation effectuée permettant d'obtenir un gel à base d'acide hyaluronique réticulé de structure dite cohésive, et
ii) de l'hydroxyapatite, à une concentration comprise entre 5% à 60% (masse/volume), ladite hydroxyapatite étant sous forme de particules de taille moyenne inférieure ou égale à 200 µm;
ladite formulation aqueuse stérile injectable possédant des propriétés viscoélastiques telles que Tanδ à la fréquence de 1 Hz est inférieur ou égal à 0.60.

2. La formulation aqueuse stérile injectable selon la revendication 1, **caractérisée en ce que** la masse moléculaire de l'acide hyaluronique, ou de l'un de ses sels, est comprise entre 2.5x10⁵ Da et 4x10⁶ Da.

3. La formulation aqueuse stérile injectable selon les revendications 1 ou 2, **caractérisée en ce que** la formulation a été stérilisée à la chaleur humide.

4. La formulation aqueuse stérile injectable selon l'une quelconque des revendications précédentes, **caractérisée en ce que** la concentration de l'acide hyaluronique réticulé, ou de l'un de ses sels, est comprise entre 1% à 3% (masse/volume), préférablement entre 1.5% à 2.5% (masse/volume).

5. La formulation aqueuse stérile injectable selon l'une quelconque des revendications précédentes, **caractérisée en ce que** la concentration de l'hydroxyapatite est comprise entre 10 à 50% (masse/volume), de préférence entre 20% à 40% (masse/volume).

6. La formulation aqueuse stérile injectable selon l'une quelconque des revendications précédentes, **caractérisée en ce que** la taille moyenne des particules d'hydroxyapatite est inférieure ou égale à 50 µm et supérieure ou égale à 10 µm.

7. La formulation aqueuse stérile injectable selon l'une quelconque des revendications précédentes, **caractérisée en ce que** la formulation comprend en outre un ou plusieurs matériaux céramiques.

8. La formulation aqueuse stérile injectable selon l'une quelconque des revendications précédentes, **caractérisée en ce que** la formulation comprend en outre un ou plusieurs anesthésiques, de préférence choisis parmi le groupe comprenant la lidocaïne seule ou en combinaison avec de l'adrénaline, la procaïne, l'étidocaïne seule ou en combinaison avec de l'adrénaline, l'articaïne seule ou en combinaison avec de l'adrénaline, la mépivacaïne, la pramocaïne, la quinisocaïne, ou un ou plusieurs des sels de ces anesthésiques.

9. La formulation aqueuse stérile injectable selon la revendication 8, **caractérisée en ce que** l'anesthésique est le chlorhydrate de lidocaine.

10. La formulation aqueuse stérile injectable selon l'une quelconque des revendications précédentes, **caractérisée en ce que** la formulation comprend en outre un ou plusieurs antioxydants.

11. La formulation aqueuse stérile injectable selon la revendication 10, **caractérisée en ce que** un ou plusieurs antioxydants est choisi parmi la famille des polyols, et de préférence choisi parmi le groupe comprenant le sorbitol, le glycérol, le mannitol ou le propylène glycol.

12. La formulation aqueuse stérile injectable selon l'une quelconque des revendications précédentes, utilisée pour le comblement et/ou la restauration de volumes et/ou le remplacement de tissus biologiques.

13. Kit contenant la formulation aqueuse stérile injectable, selon l'une quelconque des revendications 1 à 12.

14. Kit selon la revendication 13 se présentant sous forme de seringue, ampoule ou flacon.

15. Procédé de fabrication d'une formulation aqueuse stérile injectable comprenant les étapes consistant à: a) préparer un premier mélange comprenant au moins 1% à 4% en poids d'acide hyaluronique réticulé ou d'un de ses sels, par formation de liaisons covalentes entre les chaînes dudit biopolymère à l'aide de molécules bi- ou polyfonctionnelles, la réticulation effectuée permettant d'obtenir un gel à base d'acide hyaluronique réticulé de structure dite cohésive b) purifier ledit premier mélange, c) ajouter ensuite l'hydroxyapatite à une concentration comprise entre 5% à 60% (masse/volume) en la dispersant de manière homogène dans le gel à base d'acide hyaluronique réticulé, d) mettre le gel ainsi obtenu sous forme prête à l'emploi, e) stériliser le produit à la chaleur humide, ladite formulation aqueuse stérile injectable possédant des propriétés viscoélastiques telles que Tanδ à la fréquence de 1 Hz est inférieur ou égal à 0.60.

## Patentansprüche

1. Injizierbare sterile wässrige Formulierung, die für ästhetische Zwecke verwendet wird, in der Form eines teilchenhaltigen, kohäsiven viskoelastischen Gels, umfassend:
i) vernetzte Hyaluronsäure oder eines ihrer Salze in einer Konzentration zwischen 1 % und 4 % (Masse/Volumen); wobei die ausgeführte Vernetzung die Möglichkeit schafft, ein Gel zu erhalten, das auf vernetzter Hyaluronsäure mit einer sogenannten kohäsiven Struktur beruht, und
ii) Hydroxyapatit in einer Konzentration zwischen 5 % und 60 % (Masse/Volumen), wobei der Hydroxyapatit in der Form von Teilchen mit einer durchschnittlichen Größe von kleiner oder gleich 200 µm ist;
wobei die injizierbare sterile wässrige Formulierung viskoelastische Eigenschaften hat, wie zum Beispiel, dass tan δ bei der Frequenz von 1 Herz kleiner oder gleich 0,60 ist.

2. Injizierbare sterile wässrige Formulierung nach Anspruch 1, **dadurch gekennzeichnet, dass** die Molekularmasse der Hyaluronsäure oder eines ihrer Salze zwischen 2,5×10⁵ Da und 4×10⁶ Da beträgt.

3. Injizierbare sterile wässrige Formulierung nach Anspruch 1 oder 2, **dadurch gekennzeichnet, dass** die Formulierung in feuchter Wärme sterilisiert worden ist.

4. Injizierbare sterile wässrige Formulierung nach einem der vorherigen Ansprüche, **dadurch gekennzeichnet, dass** die Konzentration der vernetzten Hyaluronsäure oder eines ihrer Salze zwischen 1 % und 3 % (Masse/Volumen) beträgt, vorzugsweise zwischen 1,5 % und 2,5 % (Masse/Volumen).

5. Injizierbare sterile wässrige Formulierung nach einem der vorherigen Ansprüche, **dadurch gekennzeichnet, dass** die Konzentration von Hydroxyapatit zwischen 10 und 50 % (Masse/Volumen) beträgt, vorzugsweise zwischen 20 % und 40 % (Masse/Volumen).

6. Injizierbare sterile wässrige Formulierung nach einem der vorherigen Ansprüche, **dadurch gekennzeichnet, dass** die durchschnittliche Größe der Hydroxyapatit-Teilchen kleiner oder gleich 50 µm und größer oder gleich 10 µm ist.

7. Injizierbare sterile wässrige Formulierung nach einem der vorherigen Ansprüche, **dadurch gekennzeichnet, dass** die Formulierung ferner ein oder mehrere keramische Materialien enthält.

8. Injizierbare sterile wässrige Formulierung nach einem der vorherigen Ansprüche, **dadurch gekennzeichnet, dass** die Formulierung ferner ein oder mehrere Anästhetika enthält, die vorzugsweise ausgewählt werden aus der Gruppe, die Lidocain allein oder in Kombination mit Adrenalin, Procain, Etidocain allein oder in Kombination mit Adrenalin, Articain allein oder in Kombination mit Adrenalin, Mepivacain, Pramocain, Quinisocain oder ein oder mehrere der Salze dieser Anästhetika umfasst.

9. Injizierbare sterile wässrige Formulierung nach Anspruch 8, **dadurch gekennzeichnet, dass** das Anästhetikum Lidocainhydrochlorid ist.

10. Injizierbare sterile wässrige Formulierung nach einem der vorherigen Ansprüche, **dadurch gekennzeichnet, dass** die Formulierung ferner ein oder mehrere Antioxidantien enthält.

11. Injizierbare sterile wässrige Formulierung nach Anspruch 10, **dadurch gekennzeichnet, dass** ein oder mehrere Antioxidantien aus der Familie der Polyole gewählt werden und vorzugsweise aus der Gruppe gewählt werden, die Sorbitol, Glycerol, Mannitol oder Propylenglycol umfasst.

12. Injizierbare sterile wässrige Formulierung nach einem der vorherigen Ansprüche, die zum Füllen und/oder Wiederherstellen von Volumina und/oder Ersetzen von biologischen Geweben verwendet wird.

13. Kit, das die injizierbare sterile wässrige Formulierung nach einem der Ansprüche 1 bis 12 enthält.

14. Kit nach Anspruch 13, das die Form einer Spritze, einer Ampulle oder eines Fläschchens hat.

15. Verfahren zum Herstellen einer injizierbaren sterilen wässrigen Formulierung, das die folgenden Schritte umfasst:
a) Herstellen einer ersten Mischung, die mindestens 1 Gew.% bis 4 Gew.% von vernetzter Hyaluronsäure oder einem ihrer Salze, durch Bilden von kovalenten Bindungen zwischen den Ketten des Biopolymers mittels bi- oder polyfunktioneller Moleküle, wobei das Vernetzen ausgeführt wird durch Bereitstellen der Möglichkeit, ein Gel auf der Basis der vernetzten Hyaluronsäure mit der sogenannten kohäsiven Struktur zu erhalten, b) Reinigen der ersten Mischung, c) dann Hinzufügen von Hydroxyapatit in einer Konzentration, die zwischen 5 % und 60 % (Wasser/Volumen) liegt, durch homogenes Dispergieren desselben im Gel auf der Basis der vernetzten Hyaluronsäure, d) Überführung des so erhaltenen Gels in eine gebrauchsfertige Form, e) Sterilisieren des Produktes in feuchter Wärme, wobei die injizierbare sterile wässrige Formulierung viskoelastische Eigenschaften derart hat, dass tan δ bei der Frequenz von 1 Hz kleiner oder gleich 0,60 ist.

## Claims

1. An injectable sterile aqueous formulation, used for aesthetic purposes, in the form of a particulate cohesive viscoelastic gel comprising:
i) crosslinked hyaluronic acid, or one of its salts, at a concentration of between 1% and 4% (mass/volume); the crosslinking carried out providing the possibility of obtaining a gel based on crosslinked hyaluronic acid with a so-called cohesive structure, and
ii) hydroxyapatite at a concentration of between 5% and 60% (mass/volume), said hydroxyapatite being in the form of particles with an average size of less than or equal to 200 µm;
said injectable sterile aqueous formulation having viscoelastic properties such that tan δ at the frequency of 1 Hz is less than or equal to 0.60.

2. The injectable sterile aqueous formulation according to claim 1, **characterized in that** the molecular mass of the hyaluronic acid, or of one of its salts, is between 2.5x10⁵ Da and 4x10⁶ Da.

3. The injectable sterile aqueous formulation according to claims 1 or 2, **characterized in that** the formulation has been sterilized in humid heat.

4. The injectable sterile aqueous formulation according to any one of the preceding claims, **characterized in that** the concentration of the crosslinked hyaluronic acid or of one of its salts, is between 1% and 3% (mass/volume), preferably between 1.5% and 2.5% (mass/volume).

5. The injectable sterile aqueous formulation according to any one of the preceding claims, **characterized in that** the concentration of hydroxyapatite is between 10 and 50% (mass/volume), preferably between 20% and 40% (mass/volume).

6. The injectable sterile aqueous formulation according to any one of the preceding claims, **characterized in that** the average size of the hydroxyapatite particles is less than or equal to 50 µm and greater than or equal to 10 µm.

7. The injectable sterile aqueous formulation according to any one of the preceding claims, **characterized in that** the formulation further comprises one or several ceramic materials.

8. The injectable sterile aqueous formulation according to any one of the preceding claims, **characterized in that** the formulation further comprises one or several anesthetics preferably selected from the group comprising lidocaine alone or in combination with adrenaline, procaine, etidocaine alone or in combination with adrenaline, articaine alone or in combination with adrenaline, mepivacaine, pramocaine, quinisocaine, or one or several of the salts of these anesthetics.

9. The injectable sterile aqueous formulation according to claim 8, **characterized in that** the anaesthetic is lidocaine hydrochloride.

10. The injectable sterile aqueous formulation according to any one of the preceding claims, **characterized in that** the formulation further comprises one or several antioxidants.

11. The injectable sterile aqueous formulation according to claim 10, **characterized in that** one or several antioxidants are selected from the family of polyols, and preferably selected from the group comprising sorbitol, glycerol, mannitol or propylene glycol.

12. The injectable sterile aqueous formulation according to any one of the preceding claims, used for filling and/or restoring volumes and/or replacing biological tissues.

13. A kit containing the injectable sterile aqueous formulation according to any one of claims 1 to 12.

14. The kit according to claim 13, having the form of a syringe, an ampoule or a flask.

15. A method for preparing an injectable sterile aqueous formulation comprising the steps consisting of: a) preparing a first mixture comprising at least 1% to 4% by weight of crosslinked hyaluronic acid or of one of its salts, by forming covalent bonds between the chains of said biopolymer by means of bi- or polyfunctional molecules, the crosslinking carried out providing the possibility of obtaining a gel based on crosslinked hyaluronic acid with a so-called cohesive structure, b) purifying said first mixture, c) then adding hydroxyapatite at a concentration comprised between 5% to 60% (mass/volume) by dispersing it homogeneously in the gel based on crosslinked hyaluronic acid, d) putting the thereby obtained gel in a ready-to-use form, e) sterilizing the product in humid heat, said injectable sterile aqueous formulation having viscoelastic properties such that tan δ at the frequency of 1 Hz is less than or equal to 0.60.
